# EUROPÄISCHE PATENTSCHRIFT

(11) **EP 4 138 962 B1**
(45) Veröffentlichungstag und Bekanntmachung des Hinweises auf die Patenterteilung: **01.10.2025**
(21) Anmeldenummer: 21720275.3
(22) Anmeldetag: 22.04.2021
(51) Int. Cl.: A61M 5/315

(54) **KOLBENSTOPFEN FÜR EINE MEDIZINISCHE SPRITZE SOWIE MEDIZINISCHE SPRITZE MIT EINEM KOLBENSTOPFEN**
PLUNGER HEAD FOR A MEDICAL SYRINGE AND MEDICAL SYRINGE WITH A PLUNGER HEAD
BOUCHON DE PISTON POUR UNE SERINGUE MÉDICALE AINSI QUE SERINGUE MÉDICALE DOTÉE D'UN BOUCHON DE PISTON

(30) Priorität: 24.04.2020 EP 20171386
(43) Veröffentlichungstag der Anmeldung: 01.03.2023
(73) Patentinhaber: Fresenius Kabi Deutschland GmbH, 61352 Bad Homburg (DE)
(72) Erfinder: BRANDENBURGER, Torsten, 61440 Oberursel (DE); SCHÖNHOFEN, Michael, 61440 Oberursel (DE)
(74) Vertreter: Fresenius Kabi Deutschland GmbH
(86) Internationale Anmeldenummer: PCT/EP2021/060471
(87) Internationale Veröffentlichungsnummer: WO 2021/214194

(56) Entgegenhaltungen:
- EP-A1- 2 703 025
- EP-B1- 2 703 025

## Beschreibung

### Gebiet der Erfindung

Die Erfindung betrifft einen Kolbenstopfen für eine medizinische Spritze sowie eine medizinische Spritze, die mit dem Kolbenstopfen ausgestattet ist. Insbesondere betrifft die Erfindung eine steril verpackte medizinische Spritze, welche mit einer medizinischen Flüssigkeit, die ein Medikament enthält, vorbefüllt ist.

### Hintergrund der Erfindung

Zumindest bei vorbefüllten medizinischen Spritzen werden in der Praxis Kolben verwendet, welche einen Kolbenstopfen umfassen, der an der Kolbenstange angebracht ist.

Der Kolbenstopfen besteht aus einem Material, welches eine höhere Elastizität aufweist, als das Material der Kolbenstange.

So kann eine verbesserte Dichtwirkung bei gleichzeitig leichterem Gleiten des Kolbens erreicht werden.

Insbesondere bei arzneimittelhaltigen Spritzen sind die Anforderungen an einen derartigen Kolbenstopfen hoch. Das verwendete Material sollte möglichst keine Wechselwirkungen mit der eingefüllten medizinischen Flüssigkeit während der Lagerung eingehen. Weiter soll der Kraftaufwand zum Vorschieben des Kolbens möglichst geringgehalten werden. Mit zunehmender Kompression des Kolbenstopfens im Spritzenkörper steigt die Dichtigkeit, gleichzeitig steigen aber die erforderlichen Kräfte, um den Kolben vorzuschieben. So muss immer ein Kompromiss zwischen Dichtigkeit und Schiebekraft gewählt werden.

Problematisch ist es insbesondere, wenn es aufgrund in der Praxis immer wieder vorkommender Unterschiede der Reibkräfte um den Umfang des Kolbens zu einer Verkippung des Kolbens kommt, was die erforderliche Kraft zum weiteren Vorschieben des Kolbens deutlich erhöht.

Ein Kolbenstopfen nach dem Stand der Technik ist in Dokument EP 2 703 025 A1 offenbart.

### Aufgabe der Erfindung

Der Erfindung liegt demgegenüber die Aufgabe zugrunde, einen für eine mit einer medizinischen Flüssigkeit vorbefüllte Spritze optimal geeigneten Kolbenstopfen bereitzustellen. Insbesondere soll die Neigung des Kolbens zum Verkippen bei inhomogenen Reibkräften um seinen Umfang herum reduziert werden.

### Zusammenfassung der Erfindung

Die Aufgabe der Erfindung wird bereits durch einen Kolbenstopfen nach Anspruch 1 sowie durch eine mit dem Kolbenstopfen ausgestattete medizinische Spritze gelöst. Bevorzugte Ausführungsformen und Weiterbildungen sind dem Gegenstand der abhängigen Ansprüche, der Beschreibung sowie den Zeichnungen zu entnehmen.

Die Erfindung betrifft einen Kolbenstopfen für eine medizinische Spritze, wobei der Kolbenstopfen eine Stirnwand, eine Seitenwand sowie ein Innengewinde zur Befestigung einer Kolbenstange aufweist, wobei der Kolbenstopfen aus einem Butylkautschuk bereitgestellt ist, wobei die Stirnwand in distaler Richtung sowohl an der Außenseite als auch an der Innenseite konisch ausgebildet ist, wobei die Stirnwand zur Seitenwand hin in einen ersten Dichtring übergeht, welcher sich um den Umfang der Seitenwand erstreckt,
und wobei die Seitenwand zumindest drei weitere Dichtringe aufweist.

Es hat sich herausgestellt, dass durch die erfindungsgemäße Kombination von unterschiedlichen Maßnahmen ein Kolbenstopfen bereitgestellt werden kann, welcher optimal für eine vorbefüllte medizinische Spritze geeignet ist, insbesondere eine geringe Wechselwirkung mit eingefüllten medizinischen Flüssigkeiten hat und nur in geringem Maße zum Verkippen neigt.

Das Innengewinde dient einer exakten Führung des Kolbens. Durch den verwendeten Butylkautschuk werden geringe Wechselwirkungen mit medizinischen Flüssigkeiten sichergestellt.

Die Stirnwand ist in distaler Richtung, also in Richtung des Innenvolumens der Spritze, bei bestimmungsgemäßem Gebrauch sowohl an der Außenseite als auch an der Innenseite konisch ausgebildet. In Verbindung mit einer Kolbenstange mit einem konischen Ende, die in den Kolbenstopfen greift, wird so die durch Reibkräfte verursachte Spannung im Material an der Spitze des Kolbenstopfens konzentriert.

Das sich verformende Material verformt sich daher bei wechselnden Reibkräften eher in Richtung der Spitze zurück und neigt so weniger dazu, zur Seite hin zu verkippen.

Die Stirnwand ist vorzugsweise kegelförmig ausgebildet.

Mit dem Erreichen der Seitenwand geht die Oberseite der Stirnwand stufenlos in einen ersten Dichtring über.

Die Seitenwand beginnt hinter der Stirnwand also direkt mit einem Dichtring, welcher sich um deren Umfang erstreckt. Zwischen der Kante der Stirnwand und der Seitenwand ist also kein zylindrischer Seitenwandabschnitt vorgesehen.

Von diesem Dichtring aus erstrecken sich in proximaler Richtung zumindest drei weitere Dichtringe.

Es hat sich herausgestellt, dass durch die Verwendung von insgesamt vier Dichtringen eine besonders gute Führung bereitgestellt werden kann und eine Verkippneigung reduziert wird, ohne dass die erforderlichen Kräfte zum Vorschieben des Kolbens wesentlich steigen.

Bei einer bevorzugten Ausführungsform der Erfindung weisen die Dichtringe einen abgerundeten, insbesondere kreissegmentförmigen Querschnitt auf.

Insbesondere haben die Dichtringe einen kreissegmentförmigen Querschnitt, welcher über einen Radius oder eine Fase in die Seitenwand übergeht.

Die zusammengepressten Dichtringe bilden so gegenüber beispielsweise lamellenförmig ausgebildeten Dichtringen eine relativ breite Auflagefläche, was ebenfalls die Führung verbessert.

Vorzugsweise wird als Butylkautschuk ein Halogen-Butylkautschuk, insbesondere ein Chlorobutyl oder ein Bromobutyl, verwendet.

Das Innengewinde des Kolbenstopfens ist vorzugsweise als zweizügiges Trapezgewinde ausgebildet.

Die bei einem derartigen Gewinde relativ flach verlaufenden Gewindezähne bewirken eine reduzierte Aufweitung des Kolbenstopfens beim Vorschieben.

Bei einer Weiterbildung der Erfindung umfasst der Kolbenstopfen zumindest an seiner Seitenwand und/oder Stirnwand eine Beschichtung.

Vorzugsweise sind sowohl Seitenwand als auch Stirnwand mit einer Beschichtung versehen.

Insbesondere kann der Kolbenstopfen eine Beschichtung aus Polytetrafluorethylen umfassen.

Die Beschichtung kann dabei der Reduzierung der Reibkraft als auch der Bildung einer Migrationsbarriere dienen.

Bei einer Ausführungsform der Erfindung beträgt das Verhältnis einer Wandstärke der Seitenwand zum Durchmesser d des Kolbenstopfens mehr als 0,05, vorzugsweise mehr als 0,08 und besonders bevorzugt mehr als 0,09 und/oder weniger als 0,2, bevorzugt weniger als 0,15, besonders bevorzugt weniger als 0,11.

Die Wandstärke ist auch bei Spritzen mit einem relativ kleinen Volumen, insbesondere mit einem Füllvolumen von weniger als 15 ml, relativ stark, was ebenfalls einer guten Führung dient.

Bei einer Ausführungsform der Erfindung haben der erste Dichtring und/oder die weiteren Dichtringe eine Höhe h von mehr als 0,3 mm, vorzugsweise mehr als 0,5 mm, besonders bevorzugt mehr als 0,6 mm, und/oder weniger als 1,2 mm und/oder vorzugsweise weniger als 0,9 mm, besonders bevorzugt weniger als 0,8 mm.

Es hat sich herausgestellt, dass durch eine derartige Dimensionierung in Verbindung mit einem Spritzenkörper, welcher die Dichtringe komprimiert, ein optimaler Kompromiss zwischen Dichtwirkung und notwendiger Vorschubkraft gefunden werden konnte.

Bei einer Ausführungsform der Erfindung haben alle Dichtringe dieselbe Höhe.

Bei einer anderen Ausführungsform der Erfindung können die Dichtringe auch zumindest teilweise eine unterschiedliche Höhe aufweisen.

Insbesondere kann der distal erste Dichtring eine größere Höhe aufweisen als die nachfolgenden Dichtringe.

Hierdurch wird die Dichtwirkung im unmittelbar die medizinische Flüssigkeit angrenzenden Bereich verbessert.

Die Dichtringe, die zwischen den proximal und distal äußersten Dichtringen liegen, haben vorzugsweise den gleichen Querschnitt.

Die Dichtringe können insbesondere in einer axialen Schnittansicht einen im Wesentlichen kreissegmentförmigen Querschnitt aufweisen, wobei das Kreissegment einen Winkel von über 90°, vorzugsweise über 120° einschließt. Das Kreissegment schließt vorzugsweise einen Winkel von weniger als 180°, besonders bevorzugt von weniger als 170° ein.

Bei einer Ausführungsform der Erfindung geht die proximale und/oder distale Flanke zumindest eines Dichtrings über einen Radius, der kleiner ist als der Radius des Dichtrings, in die Seitenwand über.

Vorzugsweise entspricht der proximale Spitzenwinkel der Stirnwand im Wesentlichen dem distalen Spitzenwinkel der Stirnwand. Die Stirnwand hat also innenseitig und außenseitig denselben Spitzenwinkel und dementsprechend eine etwa gleichbleibende Wandstärke.

Der proximale Spitzenwinkel und/oder der distale Spitzenwinkel liegt bzw. liegen über 100°, bevorzugt über 120°, besonders bevorzugt über 140° und/oder unter 170°, bevorzugt unter 160° und besonders bevorzugt unter 155°.

Vorzugsweise hat bei einer mit dem erfindungsgemäßen Kolbenstopfen ausgestatteten Spritze die Stirnwand des Spritzenkörpers eine im Wesentlichen korrespondierende Form. So kann eine Vollentleerung der Spritze vorgenommen werden.

Bei einer Ausführungsform der Erfindung beträgt das Verhältnis einer Zahnhöhe z des Innengewindes zu einem Durchmesser d des Kolbenstopfens über 0,04, vorzugsweise über 0,05, und/oder unter 0,15.

So wird eine hinreichende sichere Verbindung zwischen dem Gewinde der Kolbenstange und dem Kolbenstopfen erreicht.

Der Durchmesser des Kolbenstopfens kann zwischen 8 mm und 30 mm liegen. Unter dem Durchmesser des Kolbenstopfens wird sein Maximaldurchmesser, also insbesondere der Durchmesser des größten Dichtrings, verstanden.

Der Kolbenstopfen ist insbesondere für Spritzen mit einem Füllvolumen von 2,25 ml bis 50 ml ausgebildet.

Bei einer Ausführungsform der Erfindung beträgt die Wandstärke das 1 bis 2fache, vorzugsweise das 1,2 bis 1,6fache, der Wandstärke der Seitenwand.

Die Stirnwand kann also etwas dicker ausgebildet sein als die Seitenwand, hat aber eine immer noch derart ähnliche Dicke, dass eine einfache Herstellung des Kolbenstopfens ermöglicht wird.

Unter der Wandstärke der Seitenwand wird die Wandstärke ohne die Höhe der Gewindezähne und ohne die Höhe der Dichtringe verstanden.

Diese Höhen eingerechnet, bewegen sich so die Dicke der Seitenwand nebst Gewinde und Dichtring in einem ähnlichen Bereich wie die Stirnwand, was für ein gleichmäßiges Abkühlen des Kolbenstopfens führt, wenn dieser im Spritzgussverfahren hergestellt wird.

Die Steigung des Innengewindes beträgt vorzugsweise zwischen 0,4 und 0,8 mm, besonders bevorzugt zwischen 0,5 und 0,7 mm.

Die Länge des Kolbenstopfens beträgt bei einer Ausführungsform der Erfindung zwischen 8 mm und 16 mm, vorzugsweise zwischen 10 mm und 14 mm.

Unter der Länge wird die Gesamtlänge des Kolbenstopfens einschließlich der konisch ausgebildeten Spitze verstanden.

Bei einer Ausführungsform der Erfindung umfasst ein proximal erster Dichtring eine proximale Flanke, die gegenüber der distalen Flanke abgeflacht ist.

Der Dichtring umfasst insbesondere einen kreissegmentförmigen Querschnitt, welcher auf der distalen Flanke in eine Tangente des Kreises übergeht.

Eine derartige Ausgestaltung kann bei einer Verformung zu einer höheren Steifigkeit des hinteren Dichtrings führen.

Der hintere Dichtring läuft so dem in distaler Richtung folgenden Dichtring beim Vorschieben quasi hinterher, was wiederum die Neigung zum Verkippen reduziert.

Die Erfindung betrifft des Weiteren eine Spritze mit einem Kolbenstopfen, wie er vorstehend beschrieben wurde.

Insbesondere umfasst die Spritze eine Kolbenstange mit einem distalen konischen Ende, welches an der konisch ausgebildeten Innenseite der Stirnwand des Kolbenstopfens anliegt.

Vorzugsweise ist die Spritze mit einer medizinischen Flüssigkeit, insbesondere mit einer medizinischen Flüssigkeit, die ein Medikament enthält, befüllt und mit einem Stopfen verschlossen. Der Stopfen kann ein Originalitätssiegel umfassen.

Die Spritze kann insbesondere autoklaviert sein. Insbesondere befindet sich die Spritze in einer Außenverpackung und ist in der Außenverpackung autoklaviert.

Die Außenverpackung kann insbesondere als Aufreißbeutel aus Folie ausgebildet sein.

### Kurzbeschreibung der Zeichnungen

Der Gegenstand der Erfindung soll im Folgenden bezugnehmend auf anhand der Zeichnungen Fig. 1 bis Fig. 7 dargestellte Ausführungsbeispiele näher erläutert werden.
Fig. 1 ist eine Schnittansicht einer erfindungsgemäßen medizinischen Spritze.
Fig. 2 ist eine Seitenansicht eines Ausführungsbeispiels eines Kolbenstopfens.
Fig. 3 ist eine Schnittansicht entlang der Linie A-A der Fig. 2.
Fig. 4 bis Fig. 7 sind weitere Schnittansichten von alternativen Ausführungsbeispielen eines Kolbenstopfens.

### Detaillierte Beschreibung der Zeichnungen

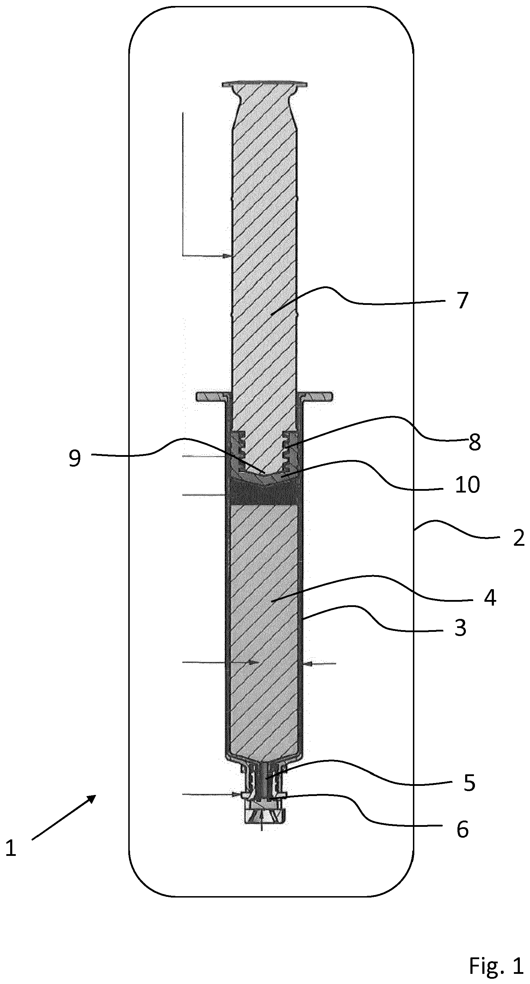

Fig. 1 zeigt in einer Schnittansicht eine medizinische Spritze 1, welche mit einem erfindungsgemäßen Kolbenstopfen ausgebildet ist.

Die Spritze 1 ist hier in der schematisch dargestellten Außenverpackung 2 angeordnet. Die Außenverpackung 2 ist als aufreißbare Folienverpackung ausgebildet und die Spritze 1 wurde in der Außenverpackung 2 autoklaviert.

Die Spritze 1 umfasst einen Spritzenkörper 3, welcher ein Volumen bereitstellt, das mit einer medizinischen Flüssigkeit 4 vorbefüllt ist. Die Düse 5 der Spritze 1 ist mit einem Stopfen 6 verschlossen.

Das Innenvolumen der Spritze 1 kann neben der medizinischen Flüssigkeit 4 ein Gas, insbesondere Luft oder Stickstoff, enthalten, um bei Druckschwankungen Verformungen des Spritzenkörpers 3 zu vermeiden.

Um die medizinische Flüssigkeit 4 nach Entfernung des Stopfens 6 auszutreiben, umfasst die Spritze 1 eine Kolbenstange 7 mit einem proximalen Griffende, welche mit dem Kolbenstopfen 10 verbunden ist.

Hierzu umfasst die Kolbenstange 7 ein Außengewinde 8, welches in das Innengewinde 16 des Kolbenstopfens 10 geschraubt ist.

Das distale Ende 9 der Kolbenstange 7 ist konisch, in diesem Ausführungsbeispiel kegelförmig, ausgebildet und liegt an der entsprechend innenseitig ausgebildeten Stirnwand 11 des Kolbenstopfens 10 an.

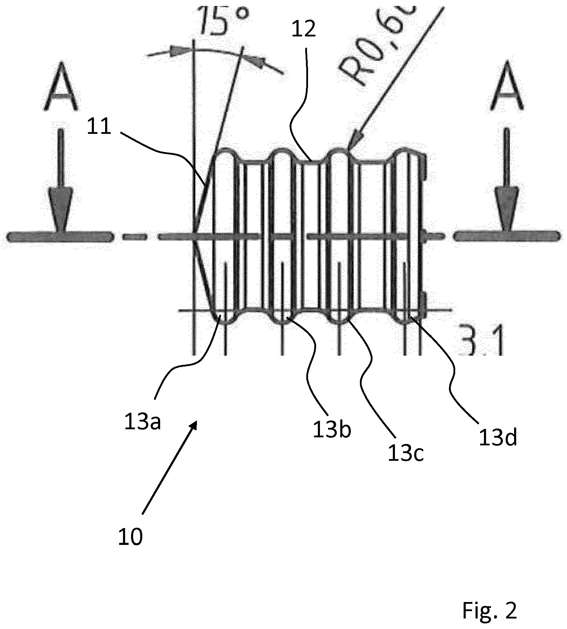

Fig. 2 ist eine Draufsicht auf die Seitenwand 12 eines erfindungsgemäßen Kolbenstopfens 10.

Die Außenkontur des Kolbenstopfens 10 ist rotationssymmetrisch ausgebildet.

Der Kolbenstopfen 10 umfasst eine kegelstumpfförmige Stirnwand 11, wobei der Kegel der Stirnwand 11 übergangslos, d.h. ohne Stufe, Nut oder ähnliches, in einen ersten Dichtring 13a übergeht.

Über die Länge des Kolbenstopfens 10 verteilt schließen sich die Dichtringe 13b bis 13d an, wobei der Dichtring 13d das proximale Ende des Kolbenstopfens 10 bildet.

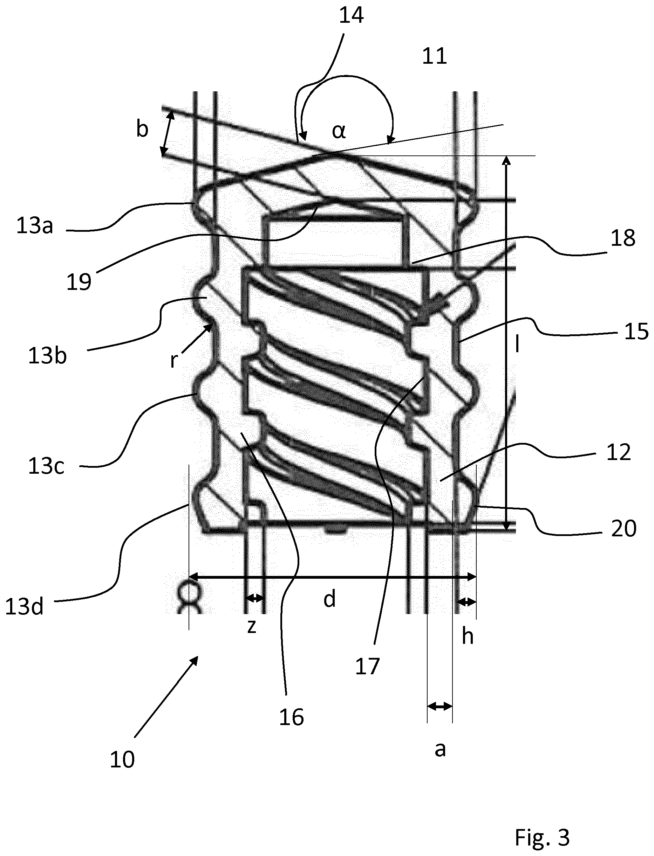

Fig. 3 ist eine Schnittansicht entlang der Linie A-A der Fig. 2, also ein axialer Schnitt entlang der Rotationsachse des Kolbenstopfens 10.

Die Stirnwand 11 ist kegelstumpfförmig ausgebildet und umfasst den Spitzenwinkel α, welcher insbesondere zwischen 140° und 160° liegen kann.

Auf der Innenseite 19 ist die Stirnwand 11 entsprechend kegelstumpfförmig ausgebildet. Mithin wird eine im Querschnitt dachförmige Ausgestaltung der Stirnwand gebildet.

Der Kolbenstopfen 10 ist mit einem Innengewinde 16 versehen, welches als zweizügiges Trapezgewinde ausgebildet ist.

Der Kolbenstopfen 10 hat einen (maximalen) Durchmesser d.

Das Innengewinde 16 hat Zähne mit einer Zahnhöhe z. Die Zähne des Innengewindes 16 können nach vorne gerichtet sein, insbesondere kann die distale Wand der Zähne einen Winkel von 2° bis 10° zur Transversalebene einnehmen.

So wird sichergestellt, dass das Außengewinde 8 der Kolbenstange 7 beim Zurückziehen des Kolbens nicht herausrutscht.

Die Wandstärke der Seitenwand a bestimmt sich definitionsgemäß ohne die Zähne des Gewindes 16 und ohne die Höhe h der Dichtringe 13a bis 13d.

Die Dichtringe 13a bis 13d sind monolithisch mit dem restlichen Kolbenstopfen ausgebildet.

Die Dichtringe 13a bis 13c sind in diesem Ausführungsbeispiel im Querschnitt im Wesentlichen kreissegmentförmig ausgebildet. Die Dichtringe 13a bis 13d können in diesem Ausführungsbeispiel einen Radius von 0,6 bis 0,7 mm aufweisen.

Die Dichtringe 13a bis 13d haben eine Höhe h zwischen 0,7 und 0,8 mm.

In diesem Ausführungsbeispiel haben die Dichtringe 13a bis 13d alle dieselbe Höhe.

Der Durchmesser d des Kolbenstopfens 10 beträgt bei diesem Ausführungsbeispiel 9 bis 9,5 mm.

Die Wandstärke der Seitenwand 12 beträgt 0,9 bis 1,0 mm.

Der proximal letzte Dichtring 13d weist gegenüber den anderen Dichtringen 13a bis 13c eine abweichende Form auf.

Die im Querschnitt kreissegmentförmige Kontur geht auf der proximalen Flanke 20 in eine Tangente über.

Diese Tangente kann zur Seitenwand 12 insbesondere einen Winkel von 60° bis 80° einschließen.

Die Länge l des Kolbenstopfens kann zwischen 10 und 14 mm liegen.

Bei diesem Ausführungsbeispiel geht das Innengewinde 16 über eine Stufe 18, welche sich zwischen dem distalen Dichtring 13a und dem folgenden Dichtring 13b befindet, in die Spitze 14 des Kolbenstopfens 10 über.

In diesem Übergangsabschnitt entspricht der Innendurchmesser des Kolbenstopfens 10 in etwa dem Durchmesser zwischen den Zähnen des Gewindes 16.

In Verbindung mit einer entsprechend geformten Kolbenstange 7 wird beim Vorschieben des Kolbenstopfens 10 erreicht, dass sich Spannungen an der Spitze 14 konzentrieren und das dahinterliegende Material des Kolbenstopfens 10 sozusagen hinterherläuft.

So wird eine optimale Kippsicherheit erreicht.

Die Wandstärke b der Stirnwand 11 ist größer als die Wandstärke der Seitenwand 12. Insbesondere ist diese 1,2 bis 1,8 mal so groß.

Zwischen den Dichtringen 13a bis 13d ist die Außenseite 15 der Seitenwand gerade ausgebildet. Die Seitenwand 12 hat in dem Bereich zwischen den Dichtringen 13a bis 13d also eine kreiszylindrische Form.

Das hier dargestellte Ausführungsbeispiel ist für Spritzen mit einem Füllvolumen von 2,25 ml vorgesehen.

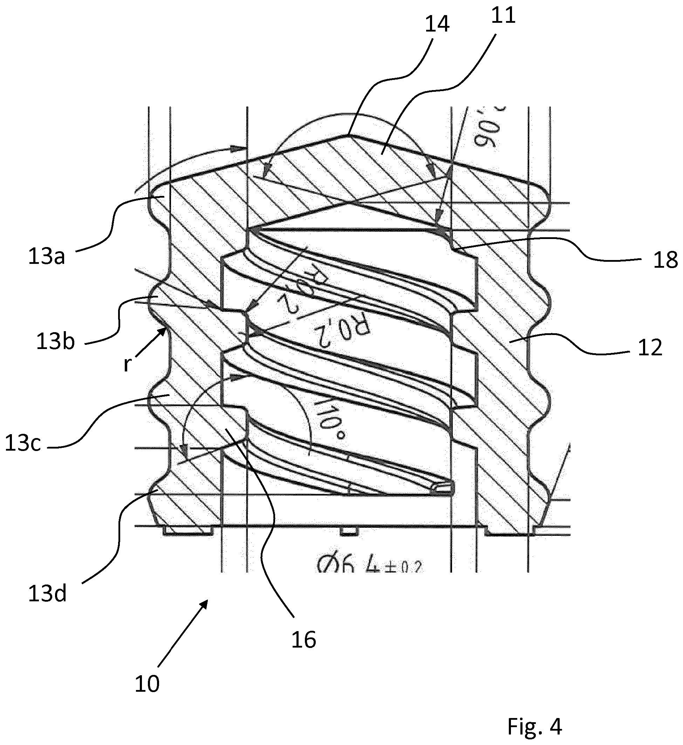

Fig. 4 bis Fig. 7 sind weitere entsprechende Schnittansichten von Ausführungsbeispielen eines Kolbenstopfens 10, wobei der Durchmesser des Kolbenstopfens jeweils größer wird.

Der in Fig. 4 dargestellte Kolbenstopfen 10 hat also einen größeren Durchmesser als der in Fig. 3 dargestellte Kolbenstopfen, insbesondere einen Durchmesser d zwischen 12 und 13 mm.

Die Dichtringe 13a bis 13c können eine der Fig. 3 entsprechende Kontur aufweisen und können insbesondere dieselben Abmessungen haben.

Im Unterschied zum Ausführungsbeispiel gemäß Fig. 3 gehen die Dichtringe 13a bis 13d über einen Radius r in die Seitenwand 12 über. Der Radius r ist in diesen Ausführungsbeispiel kleiner als der Radius des jeweiligen Kreissegments, dass den Dichtring 13 bis 13d bildet, insbesondere 0,5 bis 0,9, vorzugsweise 0,7 bis 0,8-mal so groß.

Seitenwand 12 und Stirnwand 11 sind stärker ausgebildet als beim Beispiel gemäß Fig. 3.

In diesem Ausführungsbeispiel hat die Seitenwand 12 eine Stärke bzw. Dicke a von 1,5 bis 1,7 mm und die Stirnwand 11 von 1,8 bis 2,2 mm.

Die Zähne des Gewindes 16 vergrößern sich gegenüber dem Ausführungsbeispiel gemäß Fig. 3 dagegen vorzugsweise entsprechend des Durchmessers des Kolbenstopfens 10. Diese können insbesondere eine Höhe z von 0,7 bis 0,8 mm haben.

Auch bei dem in Fig. 4 dargestellten Kolbenstopfen 10 geht das Gewinde 16 über eine Stufe 18 in die Spitze 14 über.

Das Ausführungsbeispiel gemäß Fig. 4 ist für Spritzen 1 mit einem Füllvolumen von 5 ml vorgesehen.

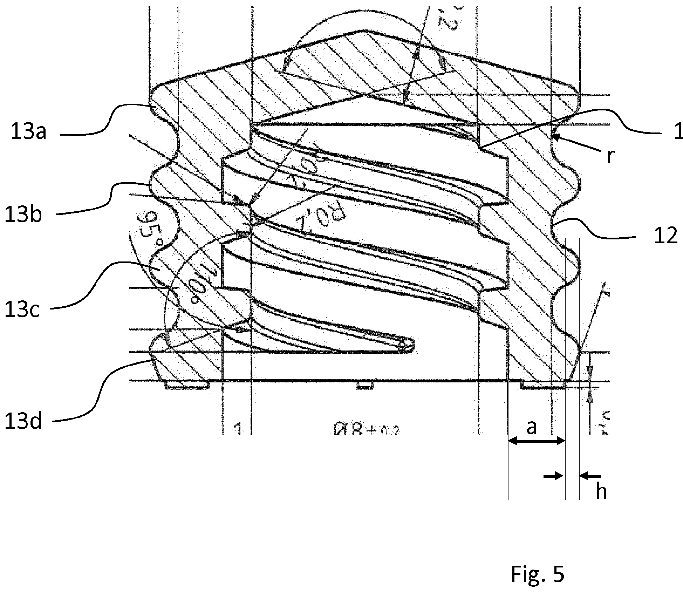

Fig. 5 zeigt ein Ausführungsbeispiel eines Kolbenstopfens mit einem Durchmesser d von 7,5 bis 8,5 mm.

Im Unterschied zu den in Fig. 3 und Fig. 4 dargestellten Ausführungsformen gehen die Dichtringe 13a bis 13d in abgerundete, insbesondere kreissegmentförmige Bereiche der Seitenwand 12 über.

Der Radius r dieser Bereiche der Seitenwand 12 kann insbesondere das 1 bis 1,5fache, vorzugsweise das 1,1 bis 1,3fache des Radius des Innenquerschnitts der Dichtringe 13a bis 13d betragen.

Zwischen den Dichtringen 13a bis 13b befinden sich mithin keine kreiszylinderförmigen Segmente der Seitenwand, sondern die Zwischenräume sind abgerundet, insbesondere kreissegmentförmig ausgebildet.

Der Radius des Innenquerschnitts der Dichtringe 13a bis 13d kann den vorangegangenen Ausführungsbeispielen entsprechen.

Insbesondere sind die Dichtringe 13a bis 13d wie bei den vorstehend genannten Ausführungsbeispielen ausgebildet.

Definitionsgemäß wird unter der Höhe h der Dichtringe 13a bis 13d die Höhe bis zum Erreichen des Wendepunkts beim Übergang zum Radius r verstanden.

Die Stärke a der Seitenwand 12 ist also durch den Radius r jeweils reduziert.

Auch die Länge l kann den Ausführungsbeispielen Fig. 3 bis Fig. 4 entsprechen.

Das hier dargestellte Ausführungsbeispiel ist insbesondere für Spritzen mit einem Füllvolumen von 10 ml vorgesehen.

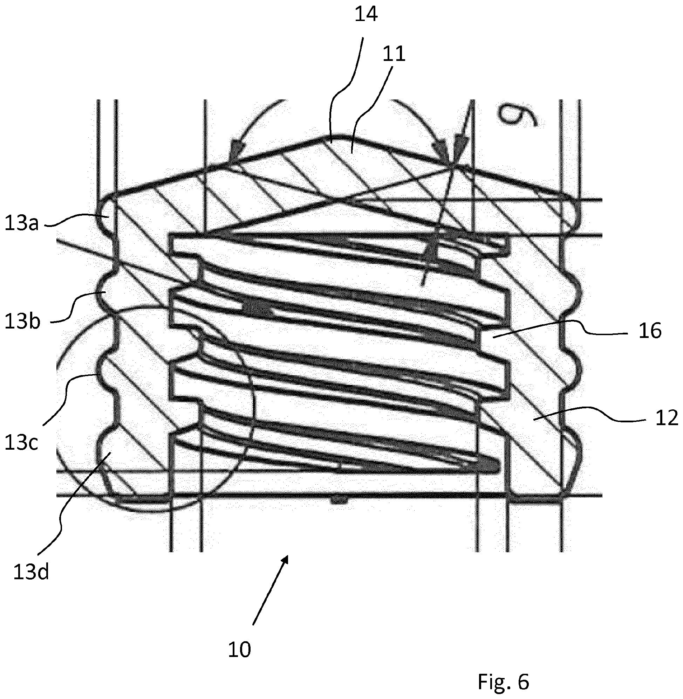

Fig. 6 zeigt eine Ausführungsform eines Kolbenstopfens 10 mit einem Durchmesser von 19,5 bis 20,5 mm.

Der Kolbenstopfen hat einen Durchmesser d von 18 bis 22 mm.

Der Innenradius des Querschnitts der Dichtringe 13a bis 13d ist etwas größer als bei den vorangegangenen Ausführungsbeispielen und kann insbesondere zwischen 0,8 und 1,0 mm liegen.

Die Seitenwand 12 ist zwischen den Dichtringen 13a bis 13d außenseitig wiederum kreiszylinderförmig ausgebildet.

Die Wandstärke der Seitenwand 12 liegt zwischen 1,8 und 2,7 mm.

Die Wandstärke der Stirnwand 11 liegt zwischen 2,4 und 2,8 mm.

Weiter geht bei diesem Ausführungsbeispiel das Gewinde 16 direkt in die Spitze 14 über.

Der hier dargestellte Kolbenstopfen 10 hat eine Länge von 12 bis 15 mm.

Der Kolbenstopfen 10 kann gemäß diesem Ausführungsbeispiel für eine Spritze 1 mit einem Füllvolumen von 20 mm vorgesehen sein.

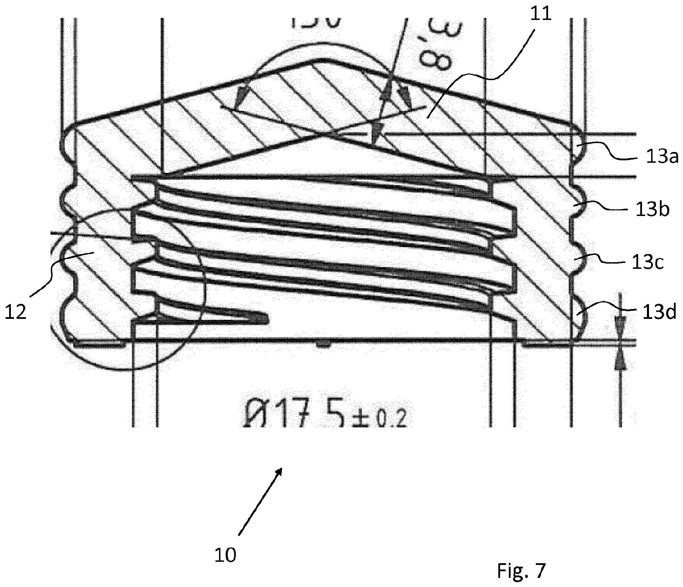

Fig. 7 zeigt eine weitere Schnittansicht eines Ausführungsbeispiels eines Kolbenstopfens 10.

Dieser hat einen Durchmesser d von 25 bis 29 mm und/oder eine Länge l von 11 bis 15 mm.

Bei diesem Ausführungsbeispiel ist der Innenradius des distal ersten Dichtrings 13a größer als die der folgenden Dichtringe 13b bis 13d.

So liegt der Innenradius des ersten Dichtrings 13a bei 1,0 bis 1,3 mm, wohingegen der Innenradius des Querschnitts der Dichtringe 13b bis 13d 0,8 bis 0,9 mm beträgt.

Die Wandstärke der Seitenwand 12 beträgt 2,8 bis 3,2 mm. Die Wandstärke der Stirnwand 11 beträgt 3,6 bis 4 mm.

Die Zähne des Gewindes 16 haben eine Höhe h zwischen 1,1 und 1,5 mm.

Der in Fig. 7 dargestellte Kolbenstopfen 10 ist insbesondere für Spritzen 1 mit einem Füllvolumen von 50 ml ausgebildet.

Durch die Erfindung konnte ein an vorbefüllte medizinische Spritzen 1 optimierter Kolbenstopfen 10 bereitgestellt werden, welcher nur eine sehr geringe Verkippneigung hat und eine gute Dichtigkeit bereitstellt.

### Bezugszeichenliste

- α: Spitzenwinkel
- a: Stärke der Seitenwand
- b: Stärke der Stirnwand
- d: Durchmesser des Kolbenstopfens
- h: Höhe der Dichtringe
- l: Länge des Kolbenstopfens
- r: Radius Übergang Dichtring zu Seitenwand
- z: Zahnhöhe des Innengewindes
- 1: Spritze
- 2: Außenverpackung
- 3: Spritzenkörper
- 4: medizinische Flüssigkeit
- 5: Düse
- 6: Stopfen
- 7: Kolbenstange
- 8: Gewinde
- 9: distales Ende der Kolbenstange
- 10: Kolbenstopfen
- 11: Stirnwand
- 12: Seitenwand
- 13a: erster Dichtring
- 13b-13d: weitere Dichtringe
- 14: Spitze
- 15: Außenseite der Außenwand
- 16: Gewinde
- 17: Gewindegrund
- 18: Stufe
- 19: Innenseite der Stirnwand
- 20: proximale Flanke

## Patentansprüche

1. Kolbenstopfen (10) für eine medizinische Spritze (1),
wobei der Kolbenstopfen (10) eine Stirnwand (11), eine Seitenwand (12) sowie ein Innengewinde (16) zur Befestigung einer Kolbenstange (7) aufweist,
wobei der Kolbenstopfen (10) aus einem Butylkautschuk bereitgestellt ist,
wobei die Stirnwand (11) in distaler Richtung sowohl an der Außenseite als auch an der Innenseite konisch ausgebildet ist,
wobei die Stirnwand (11) zur Seitenwand (12) hin übergangslos in einen ersten, distalen Dichtring (13a) übergeht, welcher sich um den Umfang der Seitenwand (12) erstreckt,
wobei die Seitenwand (12) zumindest drei weitere Dichtringe (13b-13d) aufweist,
**dadurch gekennzeichnet,**
**dass** der erste Dichtring (13a), ein zweiter Dichtring (13b) und ein dritter Dichtring (13c) im Querschnitt im Wesentlichen kreissegmentförmig ausgebildet sind und
wobei ein vierter, proximal erster Dichtring (13d) eine proximale Flanke aufweist, die gegenüber einer distalen Flanke abgeflacht ist.

2. Kolbenstopfen (10) nach dem vorstehenden Anspruch, **dadurch gekennzeichnet, dass** die Dichtringe (13a-13d) einen abgerundeten, insbesondere kreissegmentförmigen, Querschnitt aufweisen.

3. Kolbenstopfen (10) nach einem der vorstehenden Ansprüche, **dadurch gekennzeichnet, dass** der Butylkautschuk ein Halogen-Butylkautschuk, insbesondere ein Chlorobutyl oder ein Bromobutyl, ist.

4. Kolbenstopfen (10) nach einem der vorstehenden Ansprüche, **dadurch gekennzeichnet, dass** das Innengewinde (16) als zweizügiges Trapezgewinde ausgebildet ist.

5. Kolbenstopfen nach einem der vorstehenden Ansprüche,
**dadurch gekennzeichnet, dass** der Kolbenstopfen (10) eine Beschichtung zumindest an der Seitenwand (12) und/oder der Stirnwand (11) umfasst, insbesondere eine Beschichtung, die aus einem Polytetrafluorethylen gebildet wird.

6. Kolbenstopfen (10) nach einem der vorstehenden Ansprüche, **dadurch gekennzeichnet, dass** das Verhältnis einer Wandstärke der Seitenwand (12) zum Durchmesser (d) des Kolbenstopfens mehr als 0,5, vorzugsweise mehr als 0,8 und besonders bevorzugt mehr als 0,9 und/oder weniger als 2,0 bevorzugt weniger als 1,5, besonders bevorzugt weniger als 1,1 beträgt.

7. Kolbenstopfen (10) nach einem der vorstehenden Ansprüche, **dadurch gekennzeichnet, dass** ein proximaler Spitzenwinkel (α) der Stirnwand (11) im Wesentlichen einem distalen Spitzenwinkel der Stirnwand (11) entspricht.

8. Kolbenstopfen (10) nach einem der vorstehenden Ansprüche, **dadurch gekennzeichnet, dass** das Verhältnis einer Zahnhöhe (z) des Innengewindes (16) zu einem Durchmesser (d) des Kolbenstopfens (10) über 0,04, vorzugsweise über 0,05, und/oder unter 0,15 beträgt.

9. Kolbenstopfen (10) nach einem der vorstehenden Ansprüche, **dadurch gekennzeichnet, dass** die Wandstärke der Stirnwand (11) das 1 bis 2fache, vorzugsweise das 1,2 bis 1,6fache der Wandstärke der Seitenwand (12) beträgt.

10. Kolbenstopfen (10) nach einem der vorstehenden Ansprüche, **dadurch gekennzeichnet, dass** eine Steigung des Innengewindes (16) zwischen 0,4 und 0,8 mm, vorzugsweise zwischen 0,5 und 0,7 mm, beträgt.

11. Spritze (1) mit einem Kolbenstopfen (10) nach einem der vorstehenden Ansprüche.

12. Spritze (1) nach dem vorstehenden Anspruch, **dadurch gekennzeichnet, dass** die Spritze (1) eine Kolbenstange (7) mit einem distalen konischen Ende (9) ausweist, welches an der konisch ausgebildeten Innenseite der Stirnwand (11) anliegt.

13. Spritze (1) nach einem der vorstehenden Ansprüche,
**dadurch gekennzeichnet, dass** die Spitze (1) mit einer medizinischen Flüssigkeit (4), insbesondere mit einer medizinischen Flüssigkeit (4), die ein Medikament enthält, befüllt und mit einem Stopfen (6) verschlossen ist.

14. Spritze (1) nach einem der vorstehenden Ansprüche,
**dadurch gekennzeichnet, dass** die Spitze (1) autoklaviert ist, insbesondere in einer Außenverpackung (2), in welcher die Spritze (1) angeordnet ist.

## Claims

1. A plunger stopper (10) for a medical syringe (1),
wherein the plunger stopper (10) has an end wall (11), a side wall (12) and an internal thread (16) for fastening a plunger rod (7),
wherein the plunger stopper (10) is provided from a butyl rubber,
wherein the end wall (11) is conical in the distal direction both on the outside and on the inside,
wherein the end wall (11) merges seamlessly towards the side wall (12) into a first, distal sealing ring (13a), which extends around the circumference of the side wall (12),
wherein the side wall (12) has at least three further sealing rings (13b-13d),
**characterised in**
**that**
the first sealing ring (13a), a second sealing ring (13b) and a third sealing ring (13c) are substantially circular-segment-shaped in cross-section, and wherein a fourth, proximal first sealing ring (13d) has a proximal flank which is flattened relative to a distal flank.

2. The plunger stopper (10) according to the preceding claim, **characterised in that** the sealing rings (13a-13d) have a rounded, in particular circular segment-shaped, cross-section.

3. The plunger stopper (10) according to one of the preceding claims, **characterised in that** the butyl rubber is a halogenated butyl rubber, in particular a chlorobutyl or a bromobutyl.

4. The plunger stopper (10) according to one of the preceding claims, **characterised in that** the internal thread (16) is designed as a two-pass trapezoidal thread.

5. The plunger stopper according to one of the preceding claims, **characterised in that** the plunger stopper (10) comprises a coating at least on the side wall (12) and/or the end wall (11), in particular a coating which is formed from a polytetrafluoroethylene.

6. The plunger stopper (10) according to one of the preceding claims, **characterised in that** the ratio of a wall thickness of the side wall (12) to the diameter (d) of the plunger stopper is more than 0.5, preferably more than 0.8 and particularly preferably more than 0.9 and/or less than 2.0, preferably less than 1.5, particularly preferably less than 1.1.

7. The plunger stopper (10) according to one of the preceding claims, **characterised in that** a proximal tip angle (α) of the end wall (11) substantially corresponds to a distal tip angle of the end wall (11).

8. The plunger stopper (10) according to one of the preceding claims, **characterised in that** the ratio of a tooth height (z) of the internal thread (16) to a diameter (d) of the plunger stopper (10) is above 0.04, preferably above 0.05, and/or below 0.15.

9. The plunger stopper (10) according to one of the preceding claims, **characterised in that** the wall thickness of the end wall (11) is 1 to 2 times, preferably 1.2 to 1.6 times, the wall thickness of the side wall (12).

10. The plunger stopper (10) according to one of the preceding claims, **characterised in that** a pitch of the internal thread (16) is between 0.4 and 0.8 mm, preferably between 0.5 and 0.7 mm.

11. A syringe (1) with a plunger stopper (10) according to one of the preceding claims.

12. The syringe (1) according to the preceding claim, **characterised in that** the syringe (1) has a plunger rod (7) with a distal conical end (9) which bears against the conically designed inside of the end wall (11).

13. The syringe (1) according to one of the preceding claims, **characterised in that** the syringe (1) is filled with a medical fluid (4), in particular with a medical fluid (4) containing a medication, and is sealed with a stopper (6).

14. The syringe (1) according to one of the preceding claims, **characterised in that** the syringe (1) is autoclaved, in particular in an outer packaging (2) in which the syringe (1) is arranged.

## Revendications

1. Bouchon de piston (10) pour une seringue médicale (1),
dans lequel le bouchon de piston (10) présente une paroi frontale (11), une paroi latérale (12) ainsi qu'un filetage interne (16) pour la fixation d'une tige de piston (7),
dans lequel le bouchon de piston (10) est fourni à partir d'un caoutchouc butyle,
dans lequel la paroi frontale (11) est réalisée conique dans la direction distale aussi bien sur le côté externe que sur le côté interne,
dans lequel la paroi frontale (11) se transforme sans transition vers la paroi latérale (12) en une première bague d'étanchéité distale (13a) laquelle s'étend autour de la périphérie de la paroi latérale (12),
dans lequel la paroi latérale (12) présente au moins trois autres bagues d'étanchéité (13b-13d),
**caractérisé en ce**
**que**
la première bague d'étanchéité (13a), une deuxième bague d'étanchéité (13b) et une troisième bague d'étanchéité (13c) sont réalisées en section transversale essentiellement en forme de segment de cercle et dans lequel une quatrième première bague d'étanchéité proximale (13d) présente un flanc proximal qui est aplati par rapport à un flanc distal.

2. Bouchon de piston (10) selon la revendication précédente, **caractérisé en ce que** les bagues d'étanchéité (13a-13d) présentent une section transversale arrondie, en particulier en forme de segment de cercle.

3. Bouchon de piston (10) selon l'une des revendications précédentes, **caractérisé en ce que** le caoutchouc butyle est un caoutchouc halogénobutyle, en particulier un chlorobutyle ou un bromobutyle.

4. Bouchon de piston (10) selon l'une des revendications précédentes, **caractérisé en ce que** le filetage interne (16) est réalisé comme un filetage trapézoïdal à deux filets.

5. Bouchon de piston selon l'une des revendications précédentes, **caractérisé en ce que** le bouchon de piston (10) comprend un revêtement au moins sur la paroi latérale (12) et/ou la paroi frontale (11), en particulier un revêtement qui est formé à partir d'un polytétrafluoroéthylène.

6. Bouchon de piston (10) selon l'une des revendications précédentes, **caractérisé en ce que** le rapport entre une épaisseur de paroi de la paroi latérale (12) et le diamètre (d) du bouchon de piston est supérieur à 0,5, de préférence supérieur à 0,8 et de manière particulièrement préférée supérieur à 0,9 et/ou inférieur à 2,0, de préférence inférieur à 1,5, de manière particulièrement préférée inférieur à 1,1.

7. Bouchon de piston (10) selon l'une des revendications précédentes, **caractérisé en ce qu'**un angle de pointe proximal (α) de la paroi frontale (11) correspond sensiblement à un angle de pointe distal de la paroi frontale (11).

8. Bouchon de piston (10) selon l'une des revendications précédentes, **caractérisé en ce que** le rapport entre une hauteur de dent (z) du filetage interne (16) et un diamètre (d) du bouchon de piston (10) est supérieur à 0,04, de préférence supérieur à 0,05, et/ou inférieur à 0,15.

9. Bouchon de piston (10) selon l'une des revendications précédentes, **caractérisé en ce que** l'épaisseur de paroi de la paroi frontale (11) est de 1 à 2 fois, de préférence de 1,2 à 1,6 fois, l'épaisseur de paroi de la paroi latérale (12).

10. Bouchon de piston (10) selon l'une des revendications précédentes, **caractérisé en ce qu'**un pas du filetage interne (16) est compris entre 0,4 et 0,8 mm, de préférence entre 0,5 et 0,7 mm.

11. Seringue (1) comportant un bouchon de piston (10) selon l'une des revendications précédentes.

12. Seringue (1) selon la revendication précédente, **caractérisée en ce que** la seringue (1) présente une tige de piston (7) comportant une extrémité distale conique (9) laquelle s'appuie contre le côté interne conique de la paroi frontale (11).

13. Seringue (1) selon l'une des revendications précédentes, **caractérisée en ce que** la pointe (1) est remplie d'un liquide médical (4), en particulier d'un liquide médical (4) qui contient un médicament, et est fermée par un bouchon (6).

14. Seringue (1) selon l'une des revendications précédentes, **caractérisée en ce que** la pointe (1) est autoclavée, en particulier dans un emballage externe (2) dans lequel la seringue (1) est agencée.
